# EUROPEAN PATENT APPLICATION

(11) **EP 2 471 783 A1**
(43) Date of publication of application: **04.07.2012**
(21) Application number: 10382352.2
(22) Date of filing: 23.12.2010
(51) Int. Cl.: C07D 265/18, A61K 31/536, A61P 31/18

(54) **Novel polymorphic form of efavirenz**

(71) Applicant: Esteve Química, S.A., 08024 Barcelona (ES)
(72) Inventor: Ortiz Gil, Jordi, 08024 Barcelona (ES); Winter, Stephen Benedict David, 08024 Barcelona (ES); Berenguer, Maimo, Ramon, 08024 Barcelona (ES); Aguirre Ardanaz, Daniel, 08024 Barcelona (ES)
(74) Representative: ABG Patentes, S.L.

(57) **Abstract**

The present invention provides a novel crystalline form of Efavirenz, designated as form C, methods for its preparation and the use of Efavirenz form C in pharmaceutical applications, in particular in anti-HIV medicaments.

## Description

### FIELD OF THE INVENTION

The present invention relates to a novel polymorph of Efavirenz designated as Form C, processes for its preparation and pharmaceutical compositions containing it which are useful in the treatment of HIV-1 infection.

### BACKGROUND OF THE INVENTION

Efavirenz is an effective compound in the treatment of the human immunodeficiency virus (HIV) which is the retrovirus that causes progressive destruction of the human immune system with the resultant onset of AIDS. Effective treatment through inhibition of HIV reverse transcriptase has been shown for both nucleoside based inhibitors and non-nucleoside based inhibitors. Benzoxazinones, such as Efavirenz, have been found to be useful non-nucleoside based inhibitors of HIV reverse transcriptase. The chemical name of Efavirenz is (4S)-6-chloro-4-(cyclopropylethynyl)-4-(trifluoromethyl)-1,4-dihydro-2H-3,1-benzoxazin-2-one, and is represented by formula (I):

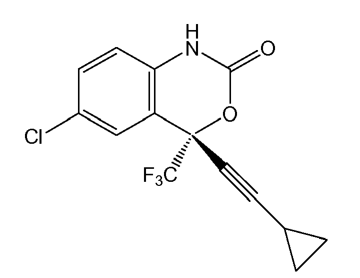

The treatment with Efavirenz may be accomplished by its use as a single compound, as a pharmaceutical composition ingredient, or in combination with other antivirals, immunomodulators, antibiotics and vaccines.

In the prior art, there are many documents describing processes for the preparation of Efavirenz such as US 5,519,021, WO94/03440, WO95/20389, US 5,698,741, WO96/37457, US 5,663,467, WO96/22955.

In addition, several solid forms of Efavirenz are known in the art. International application WO98/33782 describes crystalline polymorphic forms of Efavirenz designated as Forms I, II and III. The method for crystallizing Efavirenz uses a solvent and anti-solvent system. Form I is produced when using ethanol or methanol as a solvent and water as anti-solvent, although a slight contamination of form III in these solvent systems can convert the entire slurry to form III, which is relatively difficult to convert to form I. On the other hand, form II is obtained using 2-propanol and it can be converted to form I at low drying temperatures as low as 40°C.

Form II was previously crystallized from THF-heptane solvent system, by a crystallization procedure which required the use of high temperatures (about 90°C) to dissolve the final product. Crystals were formed by nucleation during the cooling process. This crystallization provides minimal purification and produced material with inconsistent physical properties. The final product slurry was extremely difficult to mix and handle due to its high viscosity and heterogeneous nature.

WO99/64405 discloses five crystalline forms of Efavirenz designated as forms 1, 2, 3, 4 and 5.

WO2006/018853 describes another crystalline form of Efavirenz designated as H1 as well as the amorphous form of Efavirenz.

WO2006/030299 discloses a method for the preparation of Form II of Efavirenz using solvent-antisolvent technology to improve on existing preparative methodologies that require milling of the slurry obtained after addition of water to reduce the crystal size. In addition, it refers to the conversion of form II into form I of Efavirenz by drying form II under vacuum at a temperature from about 50°C.

WO2006/040643 describes other solid forms of Efavirenz depicted as alpha, beta, gamma, gammal, gamma2, omega, delta, N, O, P and an amorphous form of Efavirenz as well as methods for their preparation.

WO2006/029079 refers to the preparation of Efavirenz and its crystallization in heptanes/ethyl acetate to prepare form 1.

WO2008/108630 discloses the crystalline forms of Efavirenz ULT-1 and ULT-2, methods for their preparation and its use in pharmaceutical applications, in particular in anti-HIV medicaments.

In view of the cited patent documents, there exist a large variety of crystalline forms of Efavirenz as well as methods for their preparation. It is generally known that different crystalline forms of an active pharmaceutical ingredient present different physico-chemical properties, such as solubility, chemical stability, melting point, density, etc., which directly influence on their production, formulation and bioavailability.

In this sense, it should be noted that Efavirenz exhibits extremely poor aqueous solubility and high intestinal permeability, making it a BSC class II drug. Therefore, its gastrointestinal absorption is limited by the dissolution rate of the drug [Sateeshkumar, S. et al., AAPS Pharm. Sci. Tech., 10(1), 81-87, 2009; Yelchuri; V.R., et al., Acta Pharm., 2010, 60, 185-195]. Thus, a polymorphic modification of Efavirenz that has improved solubility characteristics would present an advantage over less-soluble forms.

Although several polymorphic forms of Efavirenz have been described, they still show a poor solubility in water as well as in other solvents.. Therefore, there is a need for a crystalline form of Efavirenz that overcome the disadvantages mentioned above.

### BRIEF DESCRIPTION OF THE INVENTION

The authors of the present invention have surprisingly found a new polymorphic form of Efavirenz, designated as form C of Efavirenz, with an improved solubility. In particular, the form C of Efavirenz has shown an improved solubility in water/ethanol and heptane when compared to other crystalline forms of Efavirenz, as shown in examples 6 and 7 of the present application, being a very advantageous feature for its formulation and bioavailability. The fact that lower amounts of ethanol are required to dissolve Efavirenz in water makes it particularly advantageous when spray-drying technique or other techniques requiring the dissolution of this compound have to be used for formulating a pharmaceutical composition, since the presence of organic solvents is considerably reduced.

This improved solubility in water and heptane can be extrapolated to other solvents since, theoretically, the ratio of the solubility of two polymorphs at a given temperature is independent of the solvent, as the solubility is a thermodynamic invariant determined by the Gibbs energy of the forms. This leads to the conclusion that if form C is more soluble than other polymorphic form in one solvent, then it will also be more soluble in all solvents, assuming that there are no changes of polymorphic form induced by suspending in a solvent and that both forms are not solvates [Threlfall, T., Organic Process Research & Development, 2000, 4, 384-390]. Furthermore, this improved solubility positively influences on their formulation and bioavailability.

In addition, experimental tests carried out under accelerated stability conditions, in particular at 25°C and 60% relative humidity as well as at 40°C and 75% relative humidity, have shown that form C of Efavirenz is stable during at least three months. Thus, in one aspect, the present invention is directed to a polymorphic form C of Efavirenz characterized by an X-ray powder diffraction pattern which comprises characteristic 2θ values at: 7.1, 7.3, 11.0, 13.8, 14.2, 14.6, 19.1, 20.9, 21.2, 24.5 and 24.9 ± 0.2 degrees 2θ.

In another aspect, the invention relates to a method for the preparation of polymorphic form C of Efavirenz which comprises:
a) providing a solution of Efavirenz in a mixture of heptane and water, wherein the proportion of water in said mixture is between 0 and 10% by volume with respect to the total volume of the mixture;
b) heating the solution obtained in step a) at a temperature above the dissolution temperature of Efavirenz;
c) cooling the heated solution obtained in step b) at a temperature between 1 and 10°C below a curve defined when representing the following concentration / temperature values:

| Concentration (g/mL) | Temperature (°C) |
|---|---|
| 0.200 | 71 ± 3 |
| 0.067 | 56 ± 3 |
| 0.040 | 49 ± 3 |
| 0.010 | 17 ± 3 |

at the concentration of the solution provided in step a),
and alternatively:
d.1) maintaining the solution at the cooling temperature of step c) until precipitation occurs; or
d.2) seeding the solution with form C of Efavirenz to induce the precipitation of form C of Efavirenz.

Another aspect of the invention refers to a pharmaceutical composition comprising form C of Efavirenz as defined above and one or more pharmaceutically acceptable excipients.

In another aspect, the invention relates to a polymorphic form C of Efavirenz for its use as a medicament.

Finally, the invention is also directed to a polymorphic form C of Efavirenz for its use in the preparation of a medicament for the treatment of HIV.

### BRIEF DESCRIPTION OF THE FIGURES

Figure 1: X-ray powder diffractogram of Form C of Efavirenz.
Figure 2: Differential Scanning Calorimetry trace of Form C of Efavirenz.
Figure 3: Infrared spectrum of Form C of Efavirenz.
Figure 4: Solubility curve of Efavirenz in heptane
Figure 5: Comparison of the power X-ray diffraction (PXRD) pattern of Form I and Form C and monitored evolution of mixture of form I and form C to form I.
Figure 6: Comparison of the power X-ray diffraction (PXRD) pattern of Form I and Form III and monitored evolution of mixture of form I and form III to form III.
Figure 7: Dissolution profile of Form C of Efavirenz in ethanol/water
Figure 8: Solubility curves of Forms C, I and III of Efavirenz

### DETAILED DESCRIPTION OF THE INVENTION

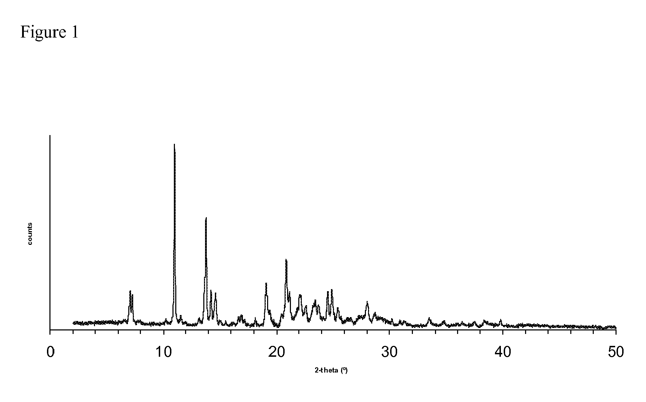

As mentioned above, the first aspect of the invention refers to a new crystalline form of Efavirenz designated as Form C. The crystalline form C of Efavirenz is characterized by an X-ray powder diffraction pattern which comprises characteristic 2θ values at: 7.1, 7.3, 11.0, 13.8, 14.2, 14.6, 19.1, 20.9, 21.2, 24.5 and 24.9 ± 0.2 degrees 2θ (Figure 1).

In a particular embodiment, the X-ray diffractogram further comprises 2θ values at 22.1, 22.6, 23.5 and 23.8 ± 0.2 degrees 2θ. More particularly, said X-ray diffractogram further comprises 2θ values at 11.5, 13.2, 25.4, 28.0 and 28.7 ± 0.2 degrees 2θ.

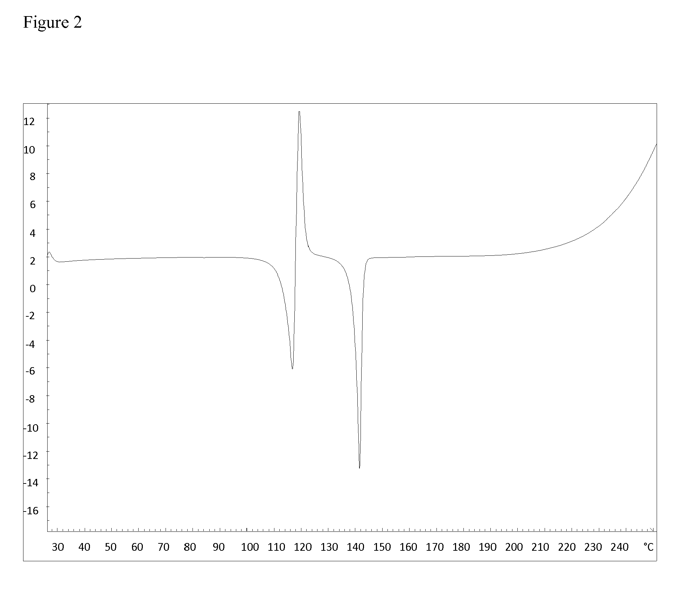

In another particular embodiment, the crystalline form C of Efavirenz is also characterized by a Differential Scanning Calorimetry thermogram having a first endothermic peak at about 115 °C measured using a gradient of about 10°C/min, immediately followed by an exotherm with a peak at about 118 °C, and then a further endotherm peak at about 140 °C (Figure 2).

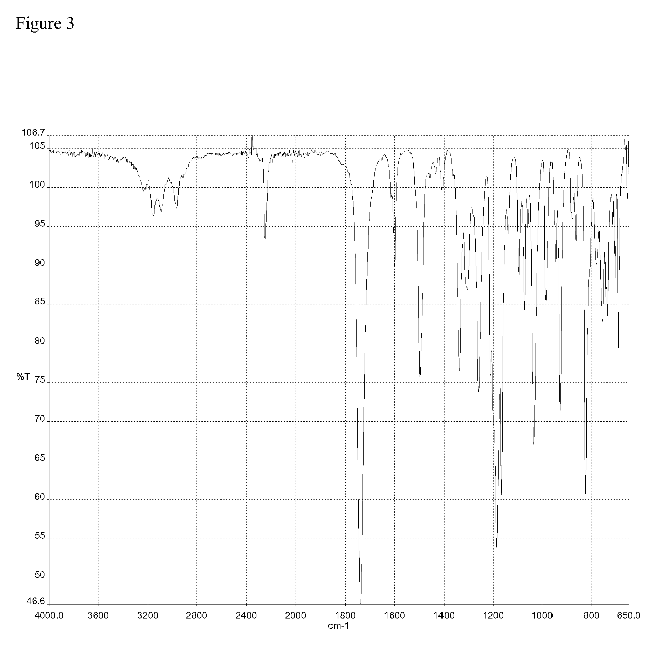

In another particular embodiment, the crystalline form C of Efavirenz is also characterized by an IR spectrum having characterizing bands at 3155.9, 2968.7, 2249.4, 1737.2, 1599.7, 1495.9, 1406.1, 1337.3, 1305.0, 1259.2, 1210.2, 1185.9, 1166.6, 1138.4, 1095.8, 1072.9, 1058.5, 1034.8, 984.9, 947.0, 929.2, 878.9, 864.7, 825.4, 780.3, 757.0, 736.4, 715.7, 706.2, 692.1 and 655.0 cm⁻¹ (Figure 3).

In another particular embodiment, the polymorphic form C is characterized by an X-ray powder diffraction pattern as set out in Figure 1.

Although the X-ray powder diffraction pattern of form C is quite similar to that obtained for polymorphic form III of Efavirenz as described in WO98/33782, experimental assays which are described in example 3 below, have pointed out that polymorph C exhibits a different behavior when compared to form III.

In particular, these assays have shown that when having a mixture of form C and form I of Efavirenz in a mixture of ethanol and water or methanol and water, the content of form C decreases with time and, after seven days, form I is basically the only polymorphic form. On the contrary, in a mixture of form III and form I of Efavirenz, the content of form I decreases with time converting it into form III. This conversion of form C into form I is not observed when form C is the only polymorphic form, i.e. when it is in its pure form, without any contamination with form I.

In a more particularly embodiment, form C of Efavirenz is characterized by one or more of:
- an X-ray powder diffraction pattern as set out in Figure 1;
- a differential scanning calorimetry thermogram as set out in Figure 2; and
- an infrared spectrum as set out in Figure 3.

The second aspect of the invention refers to a method for the preparation of polymorphic form C of Efavirenz which comprises:
a) providing a solution of Efavirenz in a mixture of heptane and water, wherein the proportion of water in said mixture is between 0 and 10% by volume with respect to the total weight of the mixture;
b) heating the solution obtained in step a) at a temperature above the dissolution temperature of Efavirenz;
c) cooling the heated solution obtained in step b) at a temperature between 1 and 10°C below a curve defined when representing the following concentration / temperature values:

| Concentration (g/mL) | Temperature (°C) |
|---|---|
| 0.200 | 71 ± 3 |
| 0.067 | 56 ± 3 |
| 0.040 | 49 ± 3 |
| 0.010 | 17 ± 3 |

at the concentration of the solution provided in step a),
and alternatively:
d.1) maintaining the solution at the cooling temperature of step c) until precipitation occurs; or
d.2) seeding the solution with form C of Efavirenz to induce the precipitation of form C of Efavirenz.

By the term "dissolution temperature" it is understood the temperature at which Efavirenz is completely dissolved in the mixture of heptane and water. This temperature depends on the concentration of Efavirenz and the proportion of water in the mixture.

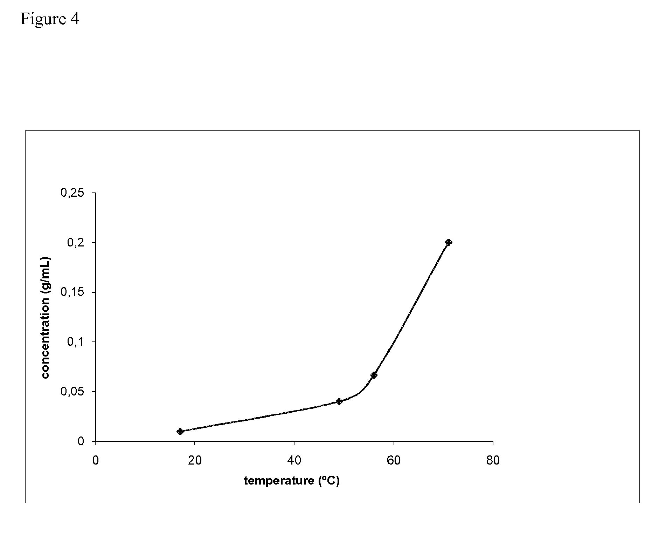

The dissolution temperature can be obtained from the solubility curve for each heptane/water proportion which is obtained when representing the concentration of Efavirenz in the particular heptane/water proportion versus the temperature at which Efavirenz is dissolved, as it is shown in Figure 4.

By using this curve, it is possible to know the dissolution temperature for every concentration of Efavirenz in a particular heptane/water mixture.

The cooling step should be done at a temperature between 1 and 10°C below the curve defined when representing the following concentration / temperature values:

| Concentration (g/mL) | Temperature (°C) |
|---|---|
| 0.200 | 71 ± 3 |
| 0.067 | 56 ± 3 |
| 0.040 | 49 ± 3 |
| 0.010 | 17 ± 3 |

at the concentration of the solution obtained in step a).

Consequently, any of steps d.1) and d.2) should also be done at said cooling temperature range defined in step c). Preferably, the cooling step is carried out at a temperature between 1 and 5°C below said curve, more preferably between 1 and 2°C below said curve.

In this sense, a skilled person, using its common general knowledge, would know how to adjust the most suitable temperature depending on the concentration of Efavirenz and the proportion of heptane/water used.

In a particular embodiment, the step d.2) of seeding is preferred.

In another particular embodiment, the water is not present in the mixture and accordingly, heptane is the only solvent used for dissolving Efavirenz in step a). In this particular embodiment, the concentration of Efavirenz is preferably not higher than 0.08 g/mL, being the temperature at which the solution is cooled in step c) below 55°C.

In another particular embodiment, the proportion of water in the mixture of heptane and water is about 1% by volume. In this particular embodiment, the concentration of Efavirenz is preferably comprised between 0.03 and 0.15 g/mL, being the temperature at which the solution is cooled in step c) between 35 and 65°C.

In a particular embodiment, the solution cooled in step c) is maintained at the temperature of cooling until precipitation of crystals of form C of Efavirenz occurs. The resulting product can be isolated by any method known in the state of the art, such as filtration, and subsequently dried.

Alternatively, the solution cooled in step c) can be seeded at the temperature of cooling with form C of Efavirenz to induce the precipitation of form C of Efavirenz. The seeding can be carried out by any seeding method known by a skilled person. The form C of Efavirenz used to seed the solution can be obtained by the process of the invention as that defined in the above paragraph. The resulting product can be isolated by any method known in the state of the art, such as filtration, and subsequently dried.

As used herein, the term "about" means a slight variation of the value specified, preferably within 10 percent of the value specified. Nevertheless, the term "about" can mean a higher tolerance of variation depending on for instance the experimental technique used. Said variations of a specified value are understood by the skilled person and are within the context of the present invention. Further, to provide a more concise description, some of the quantitative expressions given herein are not qualified with the term "about". It is understood that, whether the term "about" is used explicitly or not, every quantity given herein is meant to refer to the actual given value, and it is also meant to refer to the approximation to such given value that would reasonably be inferred based on the ordinary skill in the art, including equivalents and approximations due to the experimental and/or measurement conditions for such given value.

The present invention further relates to pharmaceutical formulations comprising the novel crystalline form C of Efavirenz. Pharmaceutical formulations of the present invention contain the crystal form according to the present invention as active ingredient, optionally in a mixture with other crystal form(s).

The pharmaceutical formulations according to the invention may further comprise, in addition to crystalline form C of Efavirenz, additional pharmaceutical active ingredients, preferably anti-HIV agents.

In addition to the active ingredient(s), the pharmaceutical formulations of the present invention may contain one or more excipients which ordinarily lack pharmaceutical activity, but have various useful properties which may, for example, enhance the stability, sterility, bioavailability, and ease of formulation of a pharmaceutical composition. These excipients are pharmaceutically acceptable, meaning that they are not harmful to humans or animals when taken appropriately and are compatible with the other ingredients in a given formulation. The excipients may be solid, semi-solid, or liquid, and may be formulated with the compound in bulk, but ultimately in the form of a unit-dose formulation (i.e., a physically discrete unit containing a specific amount of active ingredient) such as a tablet or capsule.

Generally, the pharmaceutical compositions are prepared by uniformly admixing the active ingredient with liquid or solid excipients and then shaping the product into the desired form. The pharmaceutical compositions may be in the form of suspensions, solutions, elixirs, aerosols, or solid dosage forms. Because of their ease of administration, tablets and capsules represent the most advantageous oral dosage unit form, in which case solid pharmaceutical excipients are employed.

The pharmaceutical compositions are contemplated in various formulations suitable for various modes of administration, including but not limited to inhalation, oral, rectal, parenteral (including subcutaneous, intradermal, intramuscular, intravenous), implantable, intravaginal and transdermal administration. The most suitable route of administration in any given case depends on the duration of the subject's condition, the length of treatment desired, the nature and severity of the condition being treated, and the particular formulation that is being used. The formulations may be in bulk or in unit dosage form, and may be prepared by methods well known in the art for a given formulation.

The amount of active ingredient included in a unit dosage form depends on the type of formulation in which the active ingredient is presented. A pharmaceutical composition will generally contain about 0.1 % by weight to about 99% by weight of active ingredient, preferably about 1% by weight to 50% by weight for oral administration and about 0.2% by weight to about 20% by weight for parenteral administration.

Formulations suitable for oral administration include capsules (hard and soft), cachets, lozenges, syrups, suppositories, and tablets, each containing a pre-determined amount of the active compound; as a powder or granules; as a solution or a suspension in an aqueous or non-aqueous liquid; or as an oil-in-water or water-in-oil emulsion. Such formulations may be prepared by any suitable method of pharmacy that includes the step of bringing into association the active compound and a suitable excipient or excipients. Suitable excipients include but are not limited to fillers, binders, lubricants, inert diluents, surface active dispersing agents, flavorants, antioxidants, bulking and gradating agents, adsorbants, preservatives, emulsifiers, suspending and wetting agents, glidants, disintegrants, buffers and pH-adjusting agents, and colorants. Examples of excipients include celluloses, modified celluloses, cyclodextrins, starches, oils, polyols, sugar alcohols and sugars, and others. For liquid formulations sugar, sugar alcohols, ethanol, water, glycerol, and poyalkylene glycols are particularly suitable, and may also be used in solid formulations. Cyclodextrins may be particularly useful for increasing bioavailability. Formulations for oral administration may optionally include enteric coatings known in the art to prevent degradation of the formulation in the stomach and provide release of the drug in the small intestine.

Formulations suitable for buccal or sub-lingual administration include lozenges comprising the active compound in a flavored base, usually sucrose and acacia or tragacanth, although other agents are also suitable, and pastilles comprising the compound in an inert base such as gelatin and glycerin or sucrose and acacia.

Formulations suitable for parenteral administration comprise sterile aqueous and non-aqueous injection solutions of the active compound, preferably isotonic with the blood of the intended recipient. The amount of active ingredient is preferably now about 0.1% by to about 80% by weight.

These preparations may contain, among other ingredients, anti-oxidants, buffers, bacteriostats, and solutes that render the formulation isotonic with the blood of the intended recipient. Aqueous and non-aqueous sterile suspensions may include, among others, suspending and thickening agents. The formulations may be presented in unitdose or multi-dose containers, e.g., sealed capsules and vials, and may be stored in a freeze-dried or lyophilized condition requiring only the addition of the sterile liquid excipient, for example, saline or water-for-injection immediately prior to use. Extemporaneous injection solutions and suspensions may be prepared from sterile powders, granules and tablets of the kind previously described.

Formulations suitable for rectal administration are preferably presented as unit dose suppositories. These may be prepared by admixing the active compound with one or more conventional solid excipients, e.g., cocoa butter, and then shaping the resulting mixture.

Formulations suitable for transdermal delivery include ointments, creams, lotions, and oils and contain well-known pharmaceutically and cosmetically suitable ingredients. Bases for such formulations include for example alcohols, lanolin, petrolatum, paraffin, polyethylene glycol, emulsifiers, penetration enhancing agents, and oleaginous vehicles such as oils. Skin patches may also be used, typically consisting of a fabric or paper base impregnated with a suitable dose in a transdermal formulation. Formulations suitable for transdermal administration may also be delivered by iontophoresis, and typically take the form of an optionally buffered aqueous solution of the active compound.

The following examples and figures are merely illustrative of certain embodiments of the invention and cannot be considered as restricting it in any way.

### Examples

### Analytical methods

### X-ray diffraction

Powder diffraction patterns were acquired using a PANalytical, X'Pert diffractometer using CuKα-radiation. The system configured in theta-theta, transmission geometry and equipped with a 3152/63 focusing X-ray mirror and the 0.5° divergence and anti-scatter slit and 0.02 rad soller slit. The sample was loaded onto a PW3064/60 Reflection/Transmission Spinner stage. The detector was a 3018/00 PIXcel detector, equipped with 2 mm anti-scatter slit for transmission and 0.02 rad soller slit. Approximately 10-20 mg of sample was mounted between two layers of transparent film using standard sample holders. Data was collected using X'Pert Data Collector (version 2.2g) and processed with background correction against a blank cellophane sample.

### Termal analysis

DSC analyses were recorded using a Mettler Toledo DSC822e. Samples of 5 to 7 mg were prepared in a perforated aluminium capsule, and were heated under nitrogen flow (6 mL/ Min), from 25 to 250 °C at a heating rate of 10 °C/min. Data collection and evaluation were performed with STARe software.

### Infra-red spectra

IR spectra were recorded using an FT-IR Perkin Elmer Spectrum One equipped with attenuated total reflection (ATR) module.

### Example 1: Preparation of form C of Efavirenz (Alternative 1)

A glass reaction vessel equipped with magnetic PTFE-coated stirrer, condenser and programmable temperature jacket was charged with efavirenz (200 mg, purity 99.7% by HPLC peak area), heptane (6 mL), de-ionised water (0.6 mL) and heated to complete dissolution with stirring. The mixture was cooled to 35°C at 2°C/minute and maintained with stirring at this temperature for 16 hours. During this time the product had precipitated. The mixture was cooled to 10°C at 0.1°C/minute, and after 30 minutes at this temperature was filtered and the solid was dried under vacuum at ambient temperature. Analysis of the resulting product by powder x-ray diffraction gave a diffractogram essentially the same as figure 1. The corresponding peaks and associated intensities are listed in table I.

**Table 1**

| **Peak Number** | **Position [°2- Theta]** | **d-spacing [**Å**]** | **Height [counts]** | **Rel. Int. [%]** |
|---|---|---|---|---|
| 1 | 7.07 | 12.49 | 631 | 21.0 |
| 2 | 7.28 | 12.13 | 579 | 19.1 |
| 3 | 10.98 | 8.05 | 2792 | 100.0 |
| 4 | 11.52 | 7.67 | 271 | 7.8 |
| 5 | 13.22 | 6.69 | 233 | 6.4 |
| 6 | 13.79 | 6.41 | 1721 | 60.8 |
| 7 | 14.23 | 6.22 | 641 | 21.4 |
| 8 | 14.61 | 6.06 | 597 | 19.8 |
| 9 | 19.12 | 4.64 | 742 | 25.1 |
| 10 | 20.87 | 4.25 | 1101 | 38.2 |
| 11 | 21.16 | 4.20 | 618 | 20.5 |
| 12 | 22.14 | 4.01 | 573 | 18.9 |
| 13 | 22.64 | 3.92 | 420 | 13.3 |
| 14 | 23.47 | 3.79 | 496 | 16.1 |
| 15 | 23.76 | 3.74 | 429 | 13.6 |
| 16 | 24.51 | 3.63 | 624 | 20.7 |
| 17 | 24.88 | 3.58 | 659 | 22.0 |
| 18 | 25.43 | 3.50 | 384 | 12.0 |
| 19 | 28.04 | 3.18 | 474 | 15.3 |
| 20 | 28.74 | 3.10 | 316 | 9.5 |

The resulting product which corresponds to form C of Efavirenz has been also characterized by differential scanning calorimetry (Figure 2) and infra-red spectrum (Figure 3).

### Example 2: Preparation of form C of Efavirenz by seeding with form C (Alternative 2)

A glass jacketed reactor equipped with a condenser, magnetic PTFE-coated stirrer, temperature probe and connected to a re-circulating heater-chiller unit, was charged with efavirenz (5 g, purity > 99.5% by HPLC peak area) and n-heptane (70 mL). The mixture was heated until dissolution occurred at about 65°C internal temperature. This solution was filtered under positive pressure through a jacketed, heated filter unit, washing with an additional 5 mL of heptane, into a second identical jacketed reactor previously heated to 70°C. The solution was stirred, water (0.75 mL) was added, and the mixture was cooled at 1°C/minute to 50°C. At this point, with no solid precipitated, efavirenz form C (0.025 g) was added to induce crystallisation, and the mixture was cooled with stirring at 0.1°C/min to 30°C. At this point abundant precipitation was observed. The mixture was maintained at 30°C for 2 hours and then cooled to 5°C at 0.5°C/min, stirring throughout. The mixture was stirred at 5°C for one hour and 50 minutes, then filtered and washed with cold heptane (5 ml). The white solid was dried under vacuum at ambient temperature to give efavirenz form C, 4.31 g, 86% yield, purity 99.89% HPLC peak area. Analysis of the product by powder x-ray diffraction gave a diffractogram essentially the same as figure 1.

### Example 3: Comparative and Competitive Slurry Experiments with Efavirenz forms I, III and C

Mixtures of Efavirenz form I with form III or form C were prepared according to table II. The suspensions were stirred at 20°C with small PTFE coated cross-shaped magnetic stirrers and samples taken after 1, 2 and 7 days, filtered, dried at ambient temperature under vacuum for 20 minutes and analyzed by powder X-ray diffraction.

**Table II**

| | Form I | Form C | Form III | water | Methanol | Ethanol |
|---|---|---|---|---|---|---|
| | (mg) | | | (ml) | | |
| 01 | 139 | 136 | - | 2.7 | | 1.8 |
| 02 | 151 | - | 150 | 2.7 | | 1.8 |
| 03 | 150 | 151 | | 2.7 | 1.8 | |
| 04 | 150 | | 155 | 2.7 | 1.8 | |

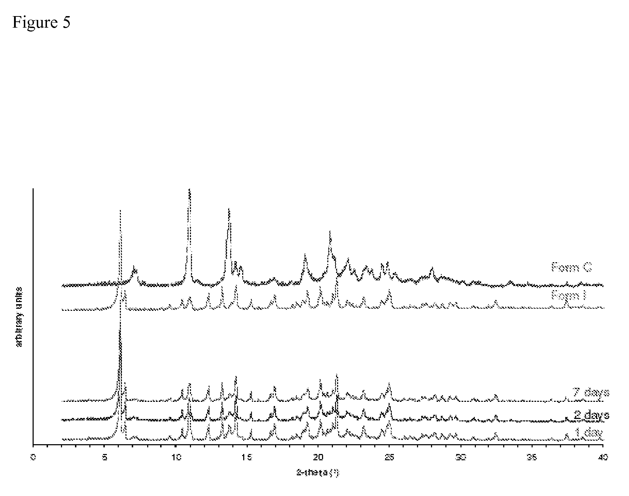

In a mixture of form I and form C, the content of form C in ethanol/water decreases with time, although some still remains in the mixture after 7 days (figure 5). In methanol/water a similar trend is observed although the transformation is slower.

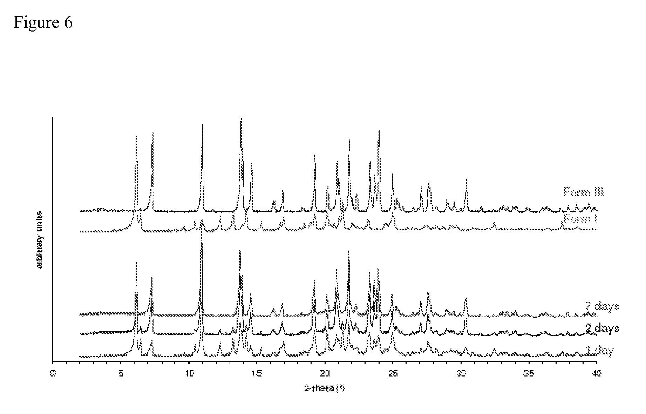

On the contrary, in a mixture of form I and form III, the content of form I in ethanol/water decreases with time, and after 7 days the mixture has converted completely to form III (Figure 6).

### Example 4: Crystallisation studies of Efavirenz Form C in heptane

Using the Radleys Metz Syn10 Multi-reactor, Efavirenz (quantity indicated in table III) was heated in heptane (5 mL) to 90°C causing complete dissolution. The solutions were cooled to the temperatures indicated in the table III at 2°C/min and then seeded with 10 mg of Efavirenz form C. This temperature was maintained for 30 minutes before cooling at 0.1°C/min to 5°C. After 16 hours at this temperature the mixture was filtered and analyzed by XRD.

**Table III: Results for crystallisation studies of form C in heptane**

| **Efavirenz (mg)** | **Concentration (g/mL)** | **Seed Temp (°C)** | **XRD result** |
|---|---|---|---|
| 53 | 0,01 | 16 | Form C |
| 152 | 0,03 | 36 | Form C |
| 250 | 0,05 | 49 | Form C |
| 350 | 0,07 | 53 | Form C |

### Example 5: Cristallisation studies of Efavirenz Form C in heptanes with 1% water

Using the Radleys Metz Syn10 Multi-reactor, Efavirenz (quantity indicated in table IV) was heated in heptane (5 mL) with 1% water to 90°C causing complete dissolution. The solutions were cooled at 2°C/min to the temperatures indicated in the table IV and then seeded with 10 mg of Efavirenz Form C. This temperature was maintained for 5 minutes before cooling at 0.1°C/min to 30°C. After 2 hours at 30°C, the mixture was cooled to 5°C at 0.1°C/min, maintained 16 hours at this temperature and then filtered and analyzed by XRD.

**Table IV: Results for crystallisation studies of form C in heptane with 1% water**

| **Efavirenz** | **Concentration (g/mL)** | **Seed Temp (°C)** | **XRD result** |
|---|---|---|---|
| 150 | 0,03 | 36 | Form C |
| 250 | 0,05 | 49 | Form C |
| 350 | 0,07 | 53 | Form C |
| 450 | 0,09 | 56 | Form C |
| 750 | 0,15 | 64 | Form C |

### Example 6. Dissolution studies of forms I, III and C in ethanol-water

Dissolution experiments were performed for forms I, III and C of Efavirenz. To an aqueous suspension of each form, ethanol was added and the volume recorded in order for dissolution. Efavirenz is hydrophobic and has a tendency to float in water. To improve the dispersion of efavirenz in water, a surfactant was used, namely, docusate sodium (DSS), the dioctyl ester of sodium sulfosuccinate. DSS is a widely used pharmaceutical excipient and food additive.

### Procedure

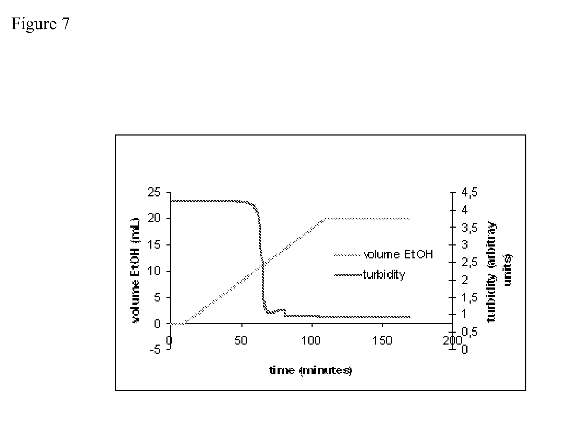

Efavirenz, polymorphic form and quantity as indicated in the table V, was suspended in water (milliQ, 20 mL) with DSS (docusate sodium, 0.02 g) in a 100 mL vessel. This mixture was placed in an ultrasonic batch for 5 minutes to break up any agglomerates and then the vessel was transferred to an automated (HEL) reaction block equipped with pitched 4-blade turbine PTFE agitators (set to 120 rpm), turbidity probes and syringe pumps. The temperature inside the vessels was controlled by jackets connected to a re-circulating heater-chiller unit. The mixtures were agitated at 20°C and turbidity probes adjusted before addition of ethanol at a continuous rate of 0.2 mL/min via the syringe pumps. The volume of ethanol required for dissolution was taken as that required for a 50% reduction in the turbidity reading. A typical dissolution profile is shown in Figure 7.

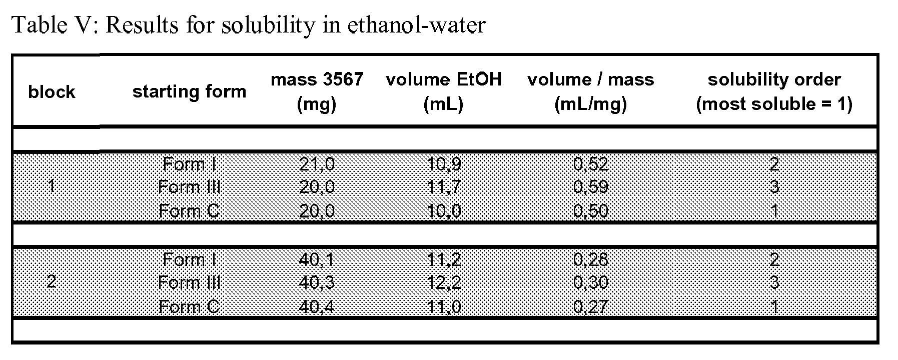

The experiments in block 2 were performed with approximately twice the amount of efavirenz, but the same volume of water, as those in block 1. Considering this clearly the relationship between mass of efavirenz and volume of ethanol required to achieve dissolution is not a linear one. That notwithstanding it is clearly seen from the results in blocks 1 and 2 that form C consistently requires less volume of ethanol than forms I and III in order to achieve dissolution, from which it can be deduced that form C shows improved solubility characteristics from the perspective of formulation of a poorly soluble product.

### Example 7. Dissolution studies of forms I, III and C in heptane

To determine the solubility curve of a given polymorphic form, different concentrations in heptane were prepared and charged to a HEL multi-reactor, using 100 mL vessels. These reactors were equipped with 4-blade PTFE turbines, (set to 150 rpm), temperature probes and turbidity probes. Temperature was controlled by applying the same re-circulating heater-chiller unit to all vessel jackets and using individual temperature compensation by external heating plates below each vessel. Heating ramps of 0.2°C/min were applied to each vessel, and the dissolution point determined by a 10% reduction in turbidity.

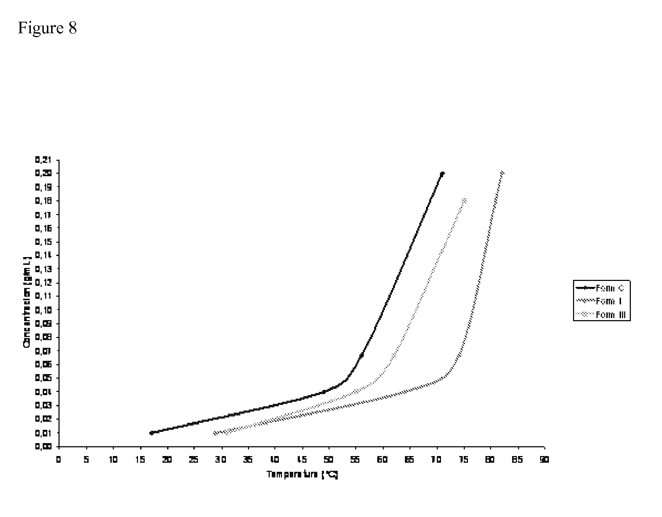

From the results (see Figure 8 and table VI) it can be deduced that form C is more soluble in heptane than forms I and III across the temperature range that was studied (17-82°C). Forms I and III have an enantiotropic relationship, being Form III less soluble at low temperature and Form I less soluble at high temperature.

**Table VI. Results for solubility in heptane**

| **Concentration** | **Dissolution Temperatures (°C)** | | |
|---|---|---|---|
| **(g/mL)** | **Form C** | **Form I** | **Form III** |
| 0,200 | 71 | 82 | - |
| 0,180 | - | - | 75 |
| | | | |
| 0,067 | 56 | 74 | 62 |
| 0,040 | 49 | 64 | 55 |
| 0,010 | 17 | 29 | 31 |

## Claims

1. A polymorphic form C of Efavirenz **characterized by** an X-ray powder diffraction pattern which comprises characteristic 2θ values at: 7.1, 7.3, 11.0, 13.8, 14.2, 14.6, 19.1, 20.9, 21.2, 24.5 and 24.9 ± 0.2 degrees 2θ.

2. The polymorphic form according to claim 1, wherein the X-ray powder diffraction pattern further comprises characteristic 2θ values at 22.1, 22.6, 23.5 and 23.8 ± 0.2 degrees 2θ.

3. The polymorphic form according to any of preceding claims, wherein the X-ray powder diffraction pattern further comprises 2θ values at 11.5, 13.2, 25.4, 28.0 and 28.7 ± 0.2 degrees 2θ.

4. The polymorphic form according to any of preceding claims, **characterized by** a Differential Scanning Calorimetry thermogram comprising a first endothermic peak at about 115 °C measured using a gradient of about 10°C/min, immediately followed by an exotherm with a peak at about 118 °C, and then a further endotherm peak at about 140 °C.

5. The polymorphic form according to any of preceding claims, **characterized by** an IR spectrum having characterizing bands at 3155.9, 2968.7, 2249.4, 1737.2, 1599.7, 1495.9, 1406.1, 1337.3, 1305.0, 1259.2, 1210.2, 1185.9, 1166.6, 1138.4, 1095.8, 1072.9, 1058.5, 1034.8, 984.9, 947.0, 929.2, 878.9, 864.7, 825.4, 780.3, 757.0, 736.4, 715.7, 706.2, 692.1 and 655.0 cm⁻¹.

6. A method for the preparation of polymorphic form C of Efavirenz which comprises:
a) providing a solution of Efavirenz in a mixture of heptane and water, wherein the proportion of water in said mixture is between 0 and 10% by volume with respect to the total volume of the mixture;
b) heating the solution obtained in step a) at a temperature above the dissolution temperature of Efvirenz;
c) cooling the heated solution obtained in step b) at a temperature between 1 and 10°C below a curve defined when representing the following concentration / temperature values:
| Concentration (g/mL) | Temperature (°C) |
|---|---|
| 0.200 | 71 ± 3 |
| 0.067 | 56 ± 3 |
| 0.040 | 49 ± 3 |
| 0.010 | 17 ± 3 |
at the concentration of the solution provided in step a),
and alternatively:
d.1) maintaining the solution at the cooling temperature of step c) until precipitation occurs; or
d.2) seeding the solution with form C of Efavirenz to induce the precipitation of form C of Efavirenz.

7. The method according to claim 6, wherein heptane is the only solvent used for dissolving Efavirenz in step a).

8. The method according to claim 7, wherein the concentration of Efavirenz is not higher than 0.08 g/mL, being the temperature at which the solution is cooled in step c) below 55°C.

9. The method according to claim 6, wherein the proportion of water in the mixture of heptane and water is about 1% by volume.

10. The method according to claim 9, wherein the concentration of Efavirenz is comprised between 0.03 and 0.15 g/mL, being the temperature at which the solution is cooled in step c) between 35 and 65°C.

11. A pharmaceutical composition comprising form C of Efavirenz as defined in any of claims 1 to 5 and one or more pharmaceutically acceptable excipients.

12. The pharmaceutical composition according to claim 11, which further comprises additional pharmaceutical anti-HIV agents.

13. Polymorphic form C of Efavirenz as defined in any of claims 1 to 5 for its use as a medicament.

14. Polymorphic form C of Efavirenz as defined in any of claims 1 to 5 for its use in the treatment of HIV.
